# EUROPEAN PATENT SPECIFICATION

(11) **EP 4 175 540 B1**
(45) Date of publication and mention of the grant of the patent: **13.03.2024**
(21) Application number: 21739592.0
(22) Date of filing: 28.06.2021
(51) Int. Cl.: A61B 5/0533, A61B 5/0531, A61B 5/00

(54) **METHOD AND APPARATUS OF ASSESSING OR MONITORING SKIN SYMPATHETIC NERVE ACTIVITY IN A LIVING SUBJECT**
VERFAHREN UND VORRICHTUNG ZUR BEURTEILUNG ODER ÜBERWACHUNG DER AKTIVITÄT SYMPATHISCHER NERVEN IN DER HAUT EINES LEBENDEN PATIENTEN
MÉTHODE ET APPAREIL D'ÉVALUATION OU DE SURVEILLANCE DE L'ACTIVITÉ DU NERF SYMPATHIQUE CUTANÉ CHEZ UN SUJET VIVANT

(30) Priority: 06.07.2020 NO 20200788
(43) Date of publication of application: 10.05.2023
(73) Proprietor: Med Storm Innovation AS, 0264 Oslo (NO)
(72) Inventor: STORM, Hanne, 0264 Oslo (NO)
(74) Representative: Onsagers AS
(86) International application number: PCT/EP2021/067616
(87) International publication number: WO 2022/008273

(56) References cited:
- EP-A1- 3 666 182
- WO-A1-2018/141850
- US-A- 3 648 686

## Description

### FIELD OF THE INVENTION

The invention relates to a method of assessing or monitoring skin sympathetic nerve activity in a living subject. The invention also relates to an associated apparatus.

### BACKGROUND OF THE INVENTION

In current clinical practice, commonly used monitoring methods to assess normal nerve activity are observation of clinical signs, skin temperature monitoring pulse amplitude monitoring in pulse oximetry plethysmography, and combinations of such monitoring methods. These methods in the background art often demonstrate an unpredictable or delayed response and other disadvantages. Therefore, it is a clinical desire to develop an objective monitoring method which is reliable, has a rapid response, and which is not affected by other disadvantages of background art. More specific, there exist no assessment tool to assess skin sympathetic nerve activity. Hence, there is a need for an improved method and apparatus of assessing skin sympathetic nerve activity in a subject, e.g. a human patient. A method according to the preamble of claim 1 is known from EP 3 666 182 A1.

### SUMMARY OF THE INVENTION

An object of the invention is to provide an improved method and apparatus of assessing or monitoring skin sympathetic nerve activity in a living subject.

The invention is set forth in the appended claims.

The present disclosure relates to a method of assessing or monitoring skin sympathetic nerve activity in a living subject, the subject having a skin. In an advantageous aspect, the subject is a human, e.g. a human patient. In an alternative aspect, the subject may be an animal.

Assessing or monitoring skin sympathetic nerve activity in the subject, e.g. the human patient, may include determining the successful of normal skin sympathetic nerve activity in a clinical setting.

An advantageous aspect relates to a method of monitoring and secure normal skin sympathetic nerve activity in the subject. An alternative aspect, relates to a method of assessing the effectiveness or injuries respectively medication or diseases may have on the skin sympathetic nerves in the subject.

The method comprises assessing or measuring electrodermal activity, wherein the electrodermal activity is chosen from the group consisting of: Skin conductance, galvanic skin response, electrodermal response, psychogalvanic reflex, skin conductance response, sympathetic skin response and skin conductance level.

In an advantageous aspect, electrodermal activity is measured. In an alternative aspect, electrodermal activity is assessed.

Advantageously, the electrodermal activity assessed or measured is skin conductance. Advantageously, the skin conductance is assessed or measured by calculating skin conductance fluctuation peaks per time unit, and when the skin conductance fluctuations peaks are present in an analyzing window with a length of about 15 to 60 seconds, the normal skin sympathetic nerve activity is assessed as being defined to not be influenced by drugs or diseases. The nerve activity obtained and assessed is successful.

Particularly advantageous, the length of the analyzing window is about 15 seconds:
The skin conductance may be assessed or measured by calculating rise time of skin conductance fluctuation, the frequency of the skin conductance fluctuations, the area under the curve of the skin conductance fluctuations, or the amplitude of the skin conductance fluctuations. In this case, when the skin conductance fluctuations are assessed in an analyzing window with a length of about 15 to 60 seconds, the normal skin sympathetic nerve activity is secured. The length of the analyzing window is advantageously about 15 to 30 seconds.

The skin conductance may be assessed or measured by calculating the number of the skin conductance fluctuation peaks per time unit (frequency). When the number of the skin conductance fluctuations is above or like of 0.13 skin conductance fluctuations per sec in an analyzing window with a length of about 15 to 60 seconds, the nerve activity is assessed and obtained and defined as normal. Particularly advantageously, the length of the analyzing window is about 15 seconds.

The skin conductance may be assessed or measured by calculating size on amplitude of the skin conductance fluctuation peaks. When the size on the amplitude is above the threshold value of 0.02 microsiemens in an analyzing window with a length of about 15 to 60 seconds, the nerve activity is assessed and obtained and defined as normal. Particularly advantageously, the length of the analyzing window is about 15 seconds.

The skin conductance may be assessed or measured by calculating area under the curve of skin conductance fluctuation peaks. When the area under the curve of skin conductance fluctuations peaks in the analyzing window with a length of about 15 to 60 seconds is above 2 microsiemensSec, the normal nerve activity is assessed and obtained and defined as normal. Particularly advantageously, the length of the analyzing window is about 15 seconds.

The skin conductance may be assessed or measured by calculating the rise time of the curve of the mean skin conductance level. When the rise time is higher or lower than 0.02 microsiemens per sec, in the analyzing window with a length of about 15 to 60 seconds is above 2, the normal nerve activity is assessed and obtained and defined as normal. Particularly advantageously, the length of the analyzing window is about 15 seconds.

The skin conductance may be assessed in the entire body of the subject, for skin sympathetic nerve activity palmar (inside the hand) and plantar (under the sole) area are advantageous.

In any of the above-mentioned methods and aspects, neural activity may be for a sympathetic nerve. However, the nerve activity may be for a mixed nerve chosen from the group consisting of motor+sympathetic, sensory+sympathetic, and motor+sensory+sympathetic.

In any of the above-mentioned methods, the normal activity may be obtained also during ongoing local analgesia or general anesthesia.

In any of the above-mentioned methods, the normal skin conductance activity may be assessed or measured at the skin level.

In any of the above-mentioned methods, the normal skin sympathetic nerve activity may be assessed or measured in the limbs including but not limted to the palmar side of the wrist, the palm, the ankle area, dorsum of the knee or the plantar part of the foot.

Any of the above-mentioned methods may further comprise the use of additional methods to assess nerve activity. Such additional methods may be chosen from the group consisting of unilateral thermometry monitoring, bilateral comparative thermometry monitoring, change in waveform amplitude in pulse oximetry plethysmography, and any combination thereof.

In any of the discloses methods, the electrodermal activity at two or more extremities may be assessed or measured, wherein in the electrodermal activity of one extremity or more extremities with or without medication or diseases are compared.

Any of the disclosed methods may further comprise stimulating electrodermal activity that disappears if there is regional anesthesia or nerve diseases.

The invention also relates to an apparatus configured to performing the disclosed method.

The apparatus may comprise a wireless sensor with Bluetooth connection to a computer or cell phone wherein a signal is processed through a computer software application and wherein the apparatus can send wireless information through a wireless technology to other computers, or mobile devices or tablets with computer software program.

The apparatus may comprise a measuring box with electrodes and computer software display on any computer tablets.

The apparatus may be used together with an accelerometer which will inform about movements to and give information about movement artefacts.

The apparatus can be connected to other methods which can assess neural block, nerve injury or normal nerve activity, wherein additional methods are chosen from the group consisting of unilateral thermometry monitoring, bilateral comparative thermometry monitoring, change in waveform amplitude in pulse oximetry plethysmography, and any combination thereof.

The apparatus can be used to assess electrodermal activity at two or more extremities to compare the extremity for normal nerve activity.

The apparatus can be used together with an electric stimulator which can be used to give information about the normal activity of the nerve, which will response during nerve stimulation.

### BRIEF DESCRIPTION OF THE DRAWINGS

Embodiments, aspects and principles of the invention will now be described in more detail with reference to the attached drawings, in which
figure 1 is a schematic flow chart illustrating a method of assessing or monitoring normal nerve activity in a subject, in a first aspect;
figure 2 is a schematic flow chart illustrating a method of assessing or monitoring normal nerve activity in a subject, in a second aspect;
figure 3 is a schematic flow chart illustrating a method of assessing or monitoring normal nerve activity in a subject, in a third aspect;
figure 4 is a schematic flow chart illustrating a method of assessing or monitoring normal nerve activity in a subject, in a third aspect;
figure 5 is a schematic block diagram illustrating an apparatus;
figure 6 is a schematic block diagram illustrating further possible aspects of the method and apparatus;
figure 7 is a schematic block diagram illustrating further possible aspects of the method and apparatus,
figure 8 is an illustration of aspects of a measurement arrangement.
figure 9 is a schematic block diagram illustrating further aspects of the apparatus,
figure 10 is a graph illustrating normal skin sympathetic nerve activity assessed by micro neurography and mirrored by skin conductance changes,

### Detailed description:

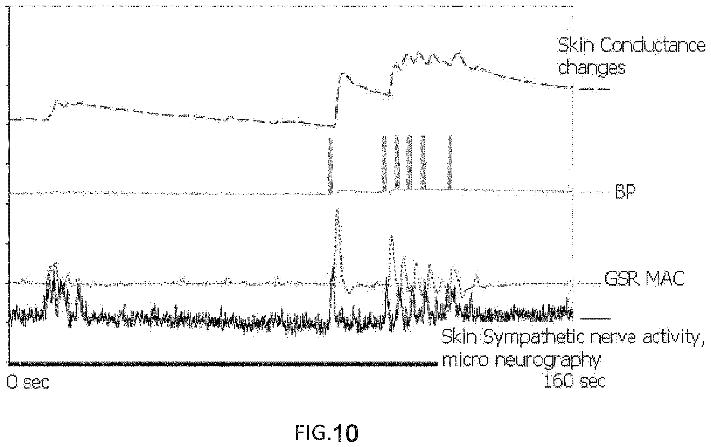

Normal nerve activity has to be secured before the skin sympathetic nerves are used for pain assessment, awakening assessment or nerve block assessment:
Normal nerve activity has to be assessed before:
1. Regional nerve block. There are three types of nerves that are bundled together within the same neural element. These nerves are sympathetic nerves, sensory nerves and motor nerves. Local anesthetic application to neural structures in the living body, e.g. the human body, creates a differential nerve blockade depending on the dose of the local anesthetic. In general, small fibers are blocked faster than those of large ones because of the time course of drug diffusion into the nerves. When e.g. a local anesthetic block is applied to the nerve, the first nerve fibers to be blocked are C- fibers including the postganglionic sympathetic fibers. C-fibers are also involved in the slow pain transmission. Myelinated fibers are blocked later than unmyelinated fibers. The smallest diameter myelinated fibers are B-fibers, also known as preganglionic sympathetic fibers are blocked next. Therefore, the first nerve fibers to be blocked by a local anesthetic nerve block are mainly sympathetic fibers including small diameter unmyelinated C-fibers postganglionic sympathetic fibers), and lightly myelinated mid sized B- fibers (pre-ganglionic sympathetic fibers), along with C fibers for slow transmission of pain.
2. Epidural anaesthesia and spinal anaesthesia may hit the skin sympathetic nerves and block these.
3. Similar for diseases, there are both nerve toxic agents and diseases developing nerve injuries or neurpathies, e.g. diabetes. Nerve injury or diseases as neuropathy may decrease the functionality in the skin sympathetic nerves, and normal nerve activity should be secured before using the skin sympathetic nerves to assess e.g. pain or awakening. If such diseases have affected the skin sympathetic nerves, the method should not be used to assess e.g. pain (number of skin conductance peaks) and awakening (area under the skin conductance peaks), illustrated in figure 10.

Based on such knowledge, the inventor has found that it is possible to monitor the sympathetic activity at the skin level by the use of a skin conductance monitor, thereby to directly monitor the normal nerve activity to secure that the nerves are without blocking agents and also without nerve diseases. This application is of extreme importance if this technology shall be used safe to assess e.g. pain and awakening. First of all, the clinician will immediately note that the skin sympathetic nerves have normal activity before these nerves are used for diagnostic purposes.

The inventor has found that electrodermal activity and skin conductance monitoring may be used to monitor and document the normal activity in the skin sympathetic nerves.

Figure 10 illustrates normal skin sympathetic nerve activity assessed by micro neurography and mirrored by skin conductance changes.

*Potential use of electrodermal activity to monitor neuromodulation techniques.*

Neuromodulation techniques including spinal cord stimulator with various stimulation modes, including but not limited to, DRG stimulation meaning dorsal root ganglion stimulation, high density stimulation, high frequency stimulation, peripheral nerve stimulation, and any other stimulation techniques used in the clinical practice and also in experiment models.

One of the mechanisms that these neuromodulation techniques provide is the sympathectomy in the limbs. There is potential use of monitoring electric-dermal activity to find out if changes in sympathetic activity in the body is achieved.

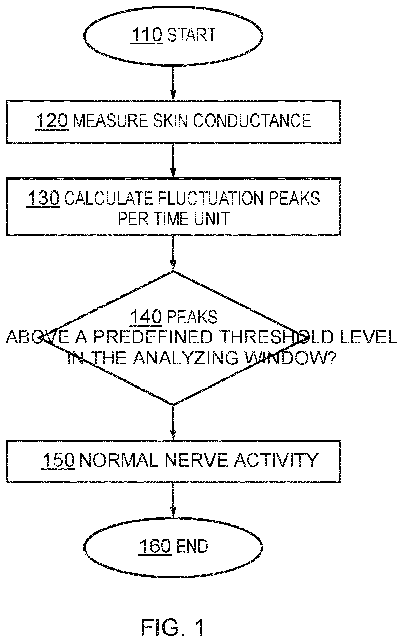

Figure 1-4 are a schematic flow charts illustrating a method of monitoring an effectiveness of a normal skin sympathetic nerve activity.

In this example, the subject is a human patient. Alternatively, the subject may be a human non-patient or an animal. In the context of this example, monitoring normal skin sympathetic nerve activity in the human patient includes determining the successful of defining the different skin conductance variables (skin conductance fluctuations per sec, amplitude of the skin conductance fluctuations, area under the skin conductance curve, the rise time of the skin conductance level):
Validation study, total of 25 recordings from volunteers were included in the analysis from the 25 volunteers recruited for this trial to secure normal skin conductance activity. The electrodes were placed 5 min on each extremity among the volunteers who were scheduled for the test. The technology is non-invasive and the test was not meant to be published, only to be used for internal purposes. Inclusion ages were 18-99 years. Exclusion criteria were defined to any injury or disease affecting the skin sympathetic nerves. After the SCM was placed, the extremities were tested for 5 min each, in randomly chosen order.

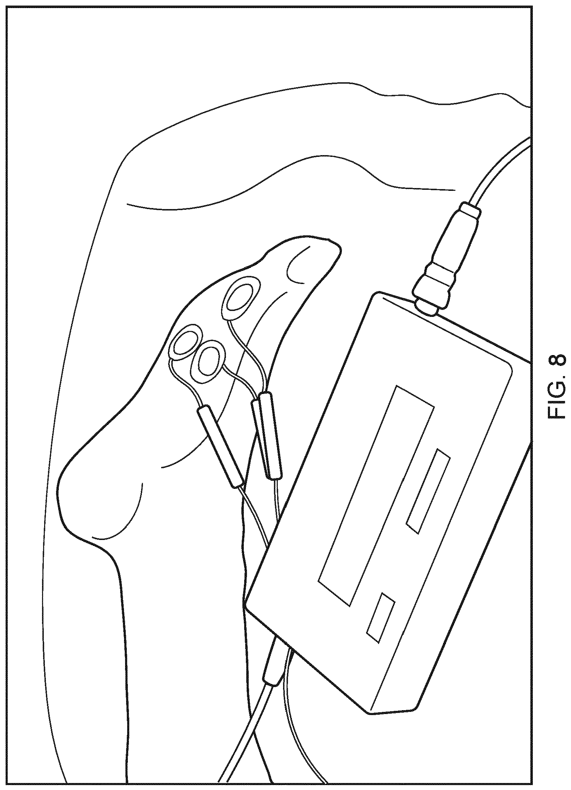

The skin conductance measurement was performed using three self-adhesive noninvasive electrodes attached to the participants' plantar (under the sole) and palmar (inside hand) for each extremity. The skin conductance responses were assessed using the SCM equipment provided by Med-Storm Innovation, Oslo, Norway, software 1.0.6.33, The SCM is a device that primarily measures changes in skin conductance real time. A skin conductance response is defined as a minimum followed by a maximum in conductance values (mS). The measurement is performed using three self-adhesive electrodes, denoted C (current), R (reference) and M (measurement) attached to plantar skin (Figure 8). The measurement unit uses the C and R electrodes in a feedback configuration to apply an exact and constant alternating voltage between the R and M electrodes. The return current from the M electrode is recorded, as its value provides direct information on the skin conductance. The recorded alternating current signal is subjected to advanced filtering which removes noise and interference before the signal is sent on to the display computer (Figure 8).

The 3-electrode system used in our study allow us to only assess skin conductance activity underneath the M electrode. The system can measure conductance values in the range 1-200mS, with a noise level (1 SD) below 0.002mS. The threshold we used to define a skin conductance response was 0.02 microsiemens.

This device has been issued a European Community declaration of conformity but not FDA approved.

Stored skin conductance recordings were analyzed, there was not found any differences between the extremities when analyzing rise time of the mean skin conductance level, number of skin conductance fluctuations per sec, area under the skin conductance curve, and amplitude of the skin conductance fluctuations. The preset skin conductance analyzing window of 15 second was used on the SCM, and the assessment lasted for 5 min for each volunteer.

Non-paired statistical tests were used to study differences between the extremities in the different volunteers, no differences were found between the extremities. The results show the following values on normal skin sympathetic nerve activity:

| | 1 minute analyzing time | 5 minute analyzing time |
|---|---|---|
| Skin conductance peaks per sec: | 0.21 +/- 0.05 | 0.19 +/- 0.04 |
| Amplitude of the skin conductance fluctuations: | 0.07 +/- 0.03 | 0.04 +/- 0.02 |
| Area under the skin conductance curve: | 5.3 +/- 1.2 | 9.6 +/- 2.2 |
| Rise time of the mean skin conductance level: | +/- 0.04 +/-0.2 | +/- 0.03 +/-0.1 |

Figure 1 is a schematic flow chart illustrating a method of monitoring an effectiveness of a normal nerve activity in a subject in a first aspect. The method starts at the initiating step 110.

The method includes the measuring step 120 of measuring electrodermal activity. Advantageously, the measured electrodermal activity is skin conductance. In alternative aspects, the electrodermal activity may be galvanic skin response, electrodermal response, psychogalvanic reflex, skin conductance response, sympathetic skin response or skin conductance level.

The method further proceeds to a calculating step 130, wherein the skin conductance measurement data are processed by calculating skin conductance fluctuation peaks per time unit.

The method further proceeds to the determining step 140. In the determining step 140, when the skin conductance fluctuations peaks are determined and counted in an analyzing window and the number of peaks is determined to be a above a predefined threshold level, the method proceeds to the establishing step 150. In establishing step 150, the normal nerve activity is established as being obtained and successful.

Advantageous, the analysis window has a length in time of about 15 to 60 seconds. Particularly advantageous, the length of the analyzing window is about 15 seconds.

When the normal nerve activity has been established to be successful in step 150, the method may be terminated at terminating step 160, or alternatively, repeated from the initiating step 110.

In the determining step 140, if the skin conductance fluctuations peaks are determined to be normal in the analyzing window, the measurement step 120, calculating step 130 and determining step 140 may be repeated.

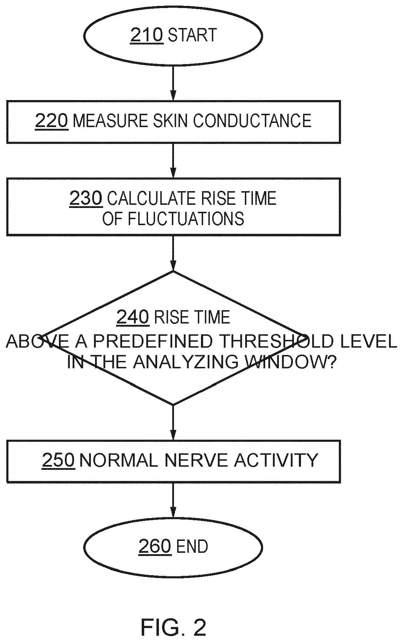

Figure 2 is a schematic flow chart illustrating a method of monitoring an effectiveness of a normal nerve activity in a subject in a second aspect. The subject is a human patient also in this example. Alternatively, the subject may be a human non-patient or an animal.

In the context of this example, monitoring an effectiveness of normal nerve activity in the human patient includes determining the successful achievement of normal nerve activity in a clinical setting.

The method starts at the initiating step 210.

The method includes the measuring step 220 of measuring electrodermal activity, wherein the measured electrodermal activity is skin conductance. In alternative aspects, the electrodermal activity could have been galvanic skin response, electrodermal response, psychogalvanic reflex, skin conductance response, sympathetic skin response or skin conductance level.

The method further proceeds to a calculating step 230, wherein a rise time of skin conductance level or fluctuations are calculated.

The method further proceeds to the determining step 240. In the determining step 240, when the rise time decreases or increases in an analyzing window according to a predefined threshold level, the method proceeds to the establishing step 250.

In establishing step 250, the normal nerve activity is established as being obtained or successful.

Advantageous, the analysis window has a length in time of about 15 to 60 seconds. Particularly advantageous, the length of the analyzing window is about 15 to 30 seconds.

When the normal nerve activity has been established to be successful in step 250, the method may be terminated at terminating step 260, or alternatively, repeated from the initiating step 210.

In the determining step 240, if the rise time of the mean skin conductance level or fluctuations do not decrease or increase, the measurement step 220, calculating step 230 and determining step 240 may be repeated (not illustrated).

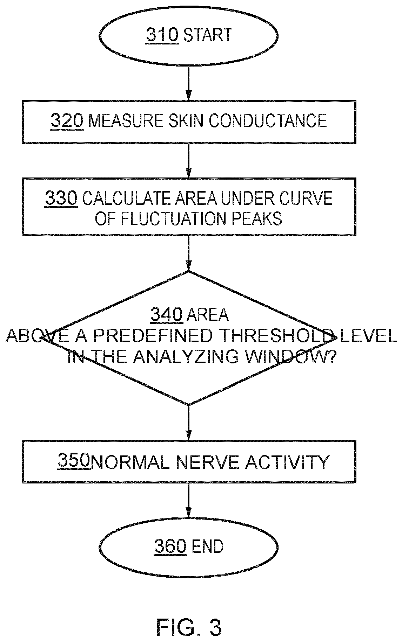

Figure 3 is a schematic flow chart illustrating a method of monitoring an effectiveness of normal nerve activity in a subject in a third aspect.

Also in this example, the subject is a human patient. Alternatively, the subject may be a human non-patient or an animal. In the context of this example, monitoring an effectiveness of normal nerve activity in the human patient includes determining the successful achievement of using the skin conductance activity in a clinical setting.

The method starts at the initiating step 310.

The method includes the measuring step 320 of measuring electrodermal activity, wherein the measured electrodermal activity is skin conductance. In alternative aspects, the electrodermal activity could have been galvanic skin response, electrodermal response, psychogalvanic reflex, skin conductance response, sympathetic skin response or skin conductance level.

The method further proceeds to a calculating step 330, wherein the area under the skin conductance fluctuations in an analyzing window are calculated.

The method further proceeds to the determining step 340. In the determining step 340, when the area under the skin conductance fluctuations increases above certain predefined threshold values in the analyzing window, the method proceeds to the establishing step 350.

In establishing step 350, the normal nerve activity is established as being obtained or successful.

Advantageous, the analysis window has a length in time of about 15 to 60 seconds. Particularly advantageous, the length of the analyzing window is about 15 seconds.

When the normal nerve activity has been established to be successful in step 350, the method may be terminated at terminating step 360, or alternatively, repeated from the initiating step 310.

In the determining step 340, if the area under the skin conductance fluctuations are above certain predefined threshold values in the analyzing window, the measurement step 320, calculating step 330 and determining step 340 may be repeated (not illustrated).

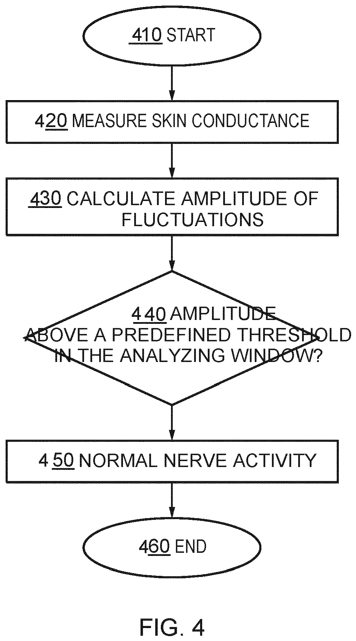

Figure 4 is a schematic flow chart illustrating a method of monitoring an effectiveness of normal nerve activity in a subject in a fourth aspect.

Also in this example, the subject is a human patient. Alternatively, the subject may be a human non-patient or an animal. In the context of this example, monitoring an effectiveness of normal nerve activity in the human patient includes determining the successful achievement of using the skin conductance activity in a clinical setting.

The method starts at the initiating step 410.

The method includes the measuring step 420 of measuring electrodermal activity, wherein the measured electrodermal activity is skin conductance. In alternative aspects, the electrodermal activity could have been galvanic skin response, electrodermal response, psychogalvanic reflex, skin conductance response, sympathetic skin response or skin conductance level.

The method further proceeds to a calculating step 430, wherein the amplitude of the skin conductance fluctuations in an analyzing window are calculated.

The method further proceeds to the determining step 440. In the determining step 340, when the amplitude of skin conductance fluctuations increases above certain predefined threshold values in the analyzing window, the method proceeds to the establishing step 450.

In establishing step 450, the normal nerve activity is established as being obtained or successful.

Advantageous, the analysis window has a length in time of about 15 to 60 seconds. Particularly advantageous, the length of the analyzing window is about 15 seconds.

When the normal nerve activity has been established to be successful in step 450, the method may be terminated at terminating step 460, or alternatively, repeated from the initiating step 410.

In the determining step 440, if the amplitude of the skin conductance fluctuations are above certain predefined threshold values in the analyzing window, the measurement step 420, calculating step 430 and determining step 440 may be repeated (not illustrated).

The step of calculating if the area under the curve of the skin conductance, rise time and amplitude of fluctuations combined with the step of calculating skin conductance fluctuation peaks per time unit, as has been described above with reference to figure 1.

In any of the methods and aspects described above with reference to figures 1, 2, 3 and/or 4, the skin conductance may be in the entire body of the subject.

In any of the methods and aspects described above with reference to figures 1, 2, 3 and/or 4, normal nerve activity for a sympathetic nerve is concluded.

In any of the methods described above with reference to figures 1, 2, 3 and/or 4, the normal nerve activity may be obtained and defined. Normal nerve activity may be defined by the subject, e.g., the human patient, in advance, i.e., not as a part of the method of assessing or monitoring the effectiveness of the skin sympathetic nerves.

In any of the methods described above with reference to figures 1, 2, 3 and/or 4, the normal nerve activity may be assessed or measured at the skin level.

In any of the methods described above with reference to figures 1, 2, 3 and/or 4, the normal nerve activity may be assessed or measured in the different location of limbs including but not limited to the palmar side of the wrist, the palm, the ankle area, dorsum of the knee, or the plantar part of the foot.

Any of the methods described above with reference to figures 1, 2, 3 and/or 4 may further comprise the use of additional methods to assess or measure normal nerve activity. Such additional methods may be chosen from the group consisting of unilateral thermometry monitoring, bilateral comparative thermometry monitoring, change in waveform amplitude in pulse oximetry plethysmography, and any combination thereof.

In any of the methods described above with reference to figures 1, 2, 3 and/or 4, the electrodermal activity, e.g, the skin conductance, at two or more extremities may be assessed or measured. In this case, the electrodermal activity, e.g. skin conductance, of one extremity with neural block or nerve injury and one extremity or more extremities without neural block or nerve injury may be compared.

Any of the disclosed methods may further comprise stimulating electrodermal activity that is activated when normal nerve activity is assessed or measured to be defined as normal. Alternatively, such stimulating of electrodermal activity may be made separately from the method, e.g., before the method is performed. In the latter case, stimulating electrodermal activity is not part of the method of assessing or monitoring the normal nerve activity in the subject. The stimulation should not be performed in the extremity where the skin conductance activity is assessed.

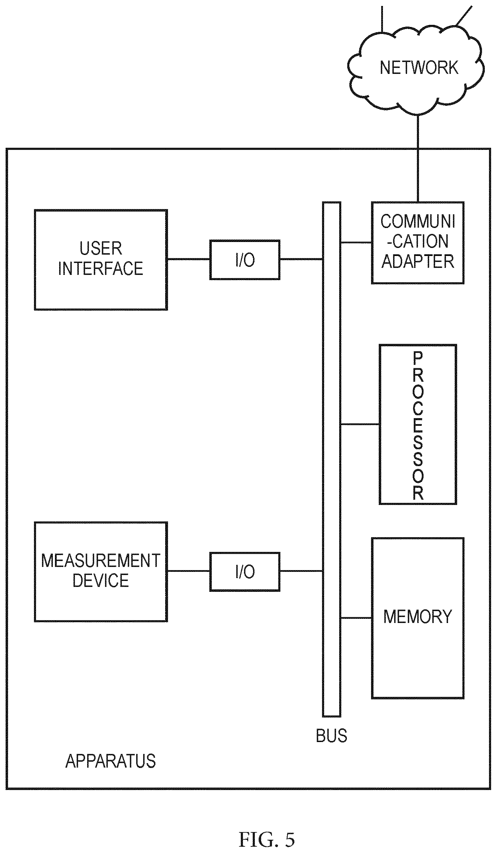

Figure 5 is a schematic block diagram illustrating an apparatus that may be used for the purpose of assessing or monitoring of the normal skin sympathetic nerve activity in a subject.

The apparatus includes an internal bus, which is interconnected to a processor, a memory, a first and a second I/O device, and optionally to a communication adapter. The communication adapter may e.g. enable communication between the apparatus and an external computer, network or system. The first I/O device is interconnected to a user interface, which enables the operation of the apparatus by a user, including providing input data to the apparatus via input devices such as a keyboard, and/or keys, switches, etc. The second I/O device is interconnected to a measurement device, which is adapted to measure electrodermal activity of a subject, in particular to measure skin conductance of an area of a human patient's skin.

The method of assessing or monitoring the normal nerve activity in the subject, e.g. the human patient, as has been disclosed in the present specification, an in particular as described above with reference to figures 1, 2, 3 and 4 above, may advantageously be implemented as a sequence of processing instructions, i.e., a computer program, that may be stored in the memory that is interconnected to the bus in the apparatus. Hence, when the processing instructions are executed by the processor in the apparatus, the apparatus will perform a method of assessing and monitoring the normal skin sympathetic nerve activity according to the present disclosure.

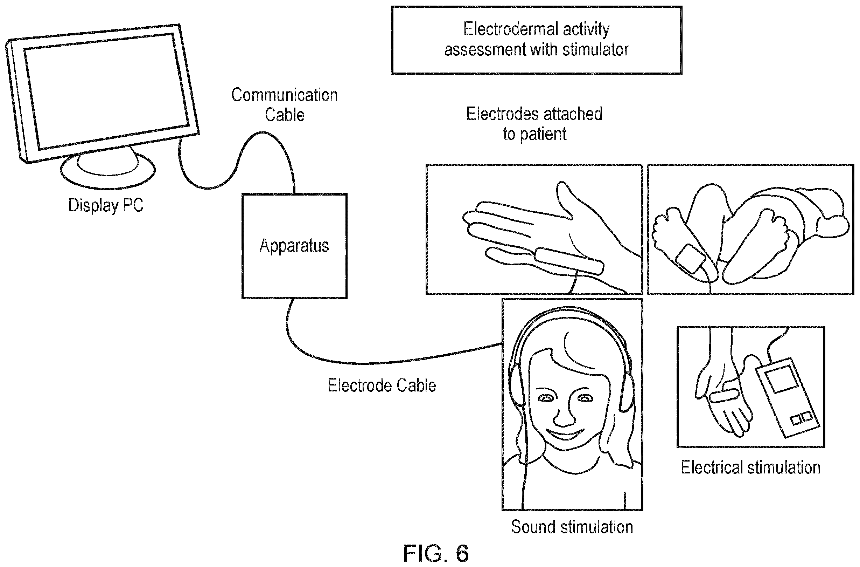

Figure 6 is a schematic block diagram illustrating further possible aspects of the method and apparatus.

As shown in figure 6, the apparatus may be interconnected to a PC with a display, for instance via a communication cable. The apparatus may also be connected to electrodes and stimulating devices via connections illustrated as electrode cable. As illustrated, the interconnected stimulating devices may include audio equipment, providing sound stimulation to the subject, and/or electrodes providing electrical stimulation to the subject. Electrodes to be attached to the subject (patient) for measuring electrodermal activity, e.g., skin conductance, have also been illustrated.

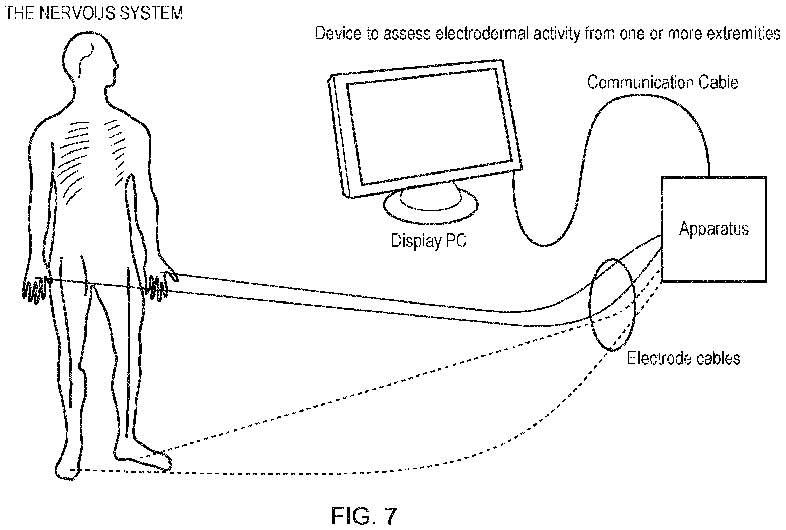

The stimulating devices may be arranged to stimulate electrodermal activity in the subject (e.g., the patient). When normal nerve activity has to be tested electrodermal activity is assessed, a stimulator to secure electrodermal responses may be used as an additional option. This stimulating device should be a sensor stimulator of a certain strength which gives rise to one or several electrodermal response(s) in the subject. It could be e.g sound, pressure, electrical, light, or smell stimulus/stimuli. Figure 7 is a schematic block diagram illustrating further possible aspects of the method and apparatus. The electrical stimulator should be on another extremity than where the skin sympathetic nerve activity is assessed.

As shown in figure 7, the apparatus is used in an arrangement to assess or monitor the normal skin sympathetic nerve activity in a subject (illustrated as the nervous system of a human), using electrodermal activity measured from a plurality of extremities.

More specifically, the features of figure 7 enables the assessment or measurement of electrodermal activity, e.g., skin conductance, at two or more extremities, wherein in the electrodermal activity of one extremity with normal nerve activity and one or more extremities which should be tested for normal skin sympathetic nerve activity.

As indicated in figure 7, separate electrodes are arranged to measure electrodermal activity, e.g. skin conductance, at the human's two hands. Optionally, as illustrated by dotted lines, electrodes may also be arranged to measure electrodermal activity, e.g. skin conductance, at the human's two feet. The electrodes are connected to the apparatus. The apparatus is further connected to a PC and display via a communication cable.

The arrangement illustrated in figure 7 provides additional features for assessment/monitoring of the effect of normal skin sympathetic nerve activity in one extremity. The extremity or extremities which not will be used for clinically assessment, will work as control extremity or extremities for the extremity which will be tested for normal nerve activity.

Figure 8 is an illustration of aspects of a measurement arrangement.

Figure 8 illustrates an extremity of a subject, namely, a foot of a human patient. Three electrodes are attached to the plantar skin of the patient's foot. The electrodes are a current (C) electrode, a reference (R) electrode, and a measurement (M) electrode, respectively. The electrodes may be self-adhesive electrodes. They are interconnected by means of electrode cables to an apparatus that may be configured to perform the method of assessing or monitoring the effectiveness of normal nerve activity in the patient, as disclosed herein.

Although a three-electrode arrangement has been shown in figure 8, it should be noted that a two electrode arrangement for measuring electrodermal activity such as electrodermal response, galvanic skin response, skin resistance or skin conductance.

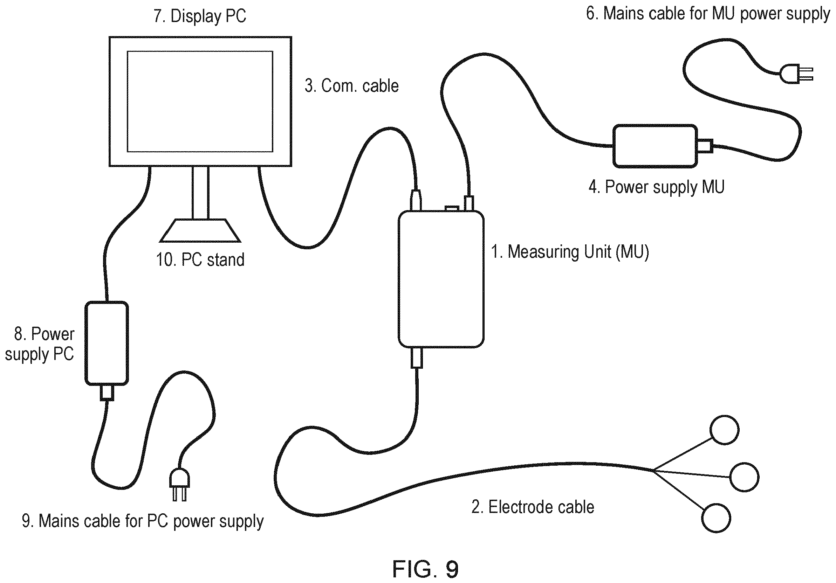

Figure 9 is a schematic block diagram illustrating further aspects of the apparatus.

Figure 9 is an overview of the apparatus and its interconnected devices during use. The apparatus 1 is interconnected with electrodes to measure electrodermal activity in the subject (e.g., patient), via electrode cable 2. The indicated arrangement of three electrodes is appropriate for measuring skin conductance on a portion of the patient's skin. The apparatus powered by a power supply 4 with a mains cable 6. A communication cable 3 interconnects the apparatus 1 with a PC 7 with a display and a stand 10. The PC may be powered by a power supply 8 with a mains cable 9.

## Claims

1. A method of assessing or monitoring the normal skin sympathetic nerve activity in a living subject, the subject having a skin, the method comprising assessing or measuring electrodermal activity,
wherein the electrodermal activity assessed or measured is skin conductance, the skin conductance is assessed or measured by calculating skin conductance fluctuation peaks per time unit, and
wherein when the number of the skin conductance fluctuations is above or equal to a predefined threshold level in an analyzing window the skin sympathetic nerve activity is assessed and obtained and defined as successful or normal,
**characterized in that** the predefined threshold level is 0.13 skin conductance fluctuations per sec.

2. The method of claim 1, wherein the skin conductance is assessed or measured by calculation the rise time of mean skin conductance level or the skin conductance fluctuations and wherein the rise time decreases or increases with a predefined threshold level in the analyzing window the skin sympathetic nerve activity is assessed and obtained and defined as successful or normal.

3. The method of claim 2, wherein the predefined threshold level is 0.02 microsiemens per time.

4. The method of claim 2, wherein the skin conductance is assessed or measured by calculating the area under the fluctuations and wherein the area has a predefined threshold level in the analyzing window the skin sympathetic nerve activity is assessed and obtained and defined as successful or normal.

5. The method of claim 4, wherein the predefined threshold is 2 microsiemensSec.

6. The method of claim 4, wherein the skin conductance is assessed or measured by calculating the amplitude of the skin conductance fluctuations, and wherein the mean amplitude of the skin conductance fluctuations has a predefined threshold level in the analyzing window the skin sympathetic nerve activity is assessed and obtained and defined as successful or normal.

7. The method of claim 6, wherein the predefined threshold level is 0.02 microsiemens.

8. The method of any claims 1 - 7 wherein the length of the analyzing window is 15 to 60 second.

9. The method of claim 8, wherein the length of the analyzing window is about 15 seconds.

10. The method of claim 1, wherein the skin conductance is measured in an entire body of the subject.

11. The method of claim 1, wherein the subject is an animal.

12. The method of claim 1, wherein the subject is a human.

13. The method of claim 1, wherein the skin sympathetic nerve activity is for a mixed nerve activity where all or some of skin conductance variables are chosen from the group consisting of skin conductance fluctuations per sec, amplitude of the skin conductance fluctuations, area under the skin conductance fluctuations and changes in rise time of the mean skin conductance level.

14. The method of claim 1, wherein the normal skin sympathetic nerve activity is obtained when the nerves are not disturbed by injuries, toxicity or nerve blocking agencies.

15. The method of claim 1, wherein the normal skin sympathetic nerves are assessed or measured at a skin level of the subject.

16. The method of claim 1, wherein the normal skin sympathetic nerve activity is assessed or measured in the subject's limbs, a palmar side of the subject's wrist, the subject's palm, the subject's ankle area, or a plantar part of the subject's foot.

17. The method of claim 1, further comprising the use of an additional process to assess or measure normal skin sympathetic nerve activity, wherein the additional process is chosen from the group consisting of unilateral thermometry monitoring, bilateral comparative thermometry monitoring, change in waveform amplitude in pulse oximetry plethysmography, and any combination thereof.

18. The method of claim 1, wherein the electrodermal activity at two or more extremities of the subject is assessed or measured, wherein in the electrodermal activity of one extremity injured or blocked nerves and one or more extremity(ies) without injured or blocked nerves are compared.

19. The method of claim 1, further comprising stimulating electrodermal activity in the subject that shows normal skin sympathetic nerve activity is assessed or measured to have been obtained or successful.

20. An apparatus **characterized in that** the apparatus is configured to performing the method of any of claims 1-19.

21. The apparatus of claim 20, wherein the apparatus comprises a wireless sensor with bluetooth connection to a computer or cell phone wherein a signal is processed through a computer software application and wherein the apparatus can send wireless information through a wireless technology to other computers, or mobile devices or tablets with computer software program.

22. The apparatus of claim 20, comprising a measuring box with electrodes and computer software display on any computer tablets.

23. The apparatus of claim 20, wherein the apparatus is configured to be used together with an accelerometer which will inform about movements to and give information about movement artefacts.

24. The apparatus of claim 20, wherein the apparatus is further configured to perform another process which can assess normal nerve activity, wherein the additional process is chosen from the group consisting of unilateral thermometry monitoring, bilateral comparative thermometry monitoring, change in waveform amplitude in pulse oximetry plethysmography, and any combination thereof.

25. The apparatus of claim 20, wherein the apparatus is configured to assess electrodermal activity at two or more extremities to compare the extremity with normal skin sympathetic nerve activity to one or more extremities without normal skin sympathetic nerve activity.

26. The apparatus of claim 20, wherein the apparatus further comprises an electrodermal activity stimulator which is configured to give information about when the normal skin sympathetic nerve activity to define that the nerve activity can be used for clinical assessment like pain and awakening.

## Patentansprüche

1. Verfahren zum Beurteilen oder Überwachen der normalen Aktivität des Sympathikus in der Haut bei einem lebenden Subjekt, wobei das Subjekt eine Haut aufweist, wobei das Verfahren Beurteilen oder Messen von elektrodermaler Aktivität umfasst,
wobei die beurteilte oder gemessene elektrodermale Aktivität Hautleitfähigkeit ist, wobei die Hautleitfähigkeit durch Berechnen von Fluktuationsspitzen der Hautleitfähigkeit pro Zeiteinheit beurteilt oder gemessen wird, und
wobei, wenn die Anzahl der Fluktuationen der Hautleitfähigkeit größer oder gleich einem vordefinierten Grenzwert in einem Analysefenster ist, die Aktivität des Sympathikus in der Haut beurteilt und erhalten und als erfolgreich oder normal definiert wird,
**dadurch gekennzeichnet, dass** der vordefinierte Grenzwert bei 0,13 Fluktuationen der Hautleitfähigkeit pro Sekunde liegt.

2. Verfahren nach Anspruch 1, wobei die Hautleitfähigkeit durch Berechnen der Anstiegszeit des mittleren Hautleitfähigkeitsniveaus oder der Fluktuationen der Hautleitfähigkeit beurteilt oder gemessen wird und wobei, wenn die Anstiegszeit mit einem vordefinierten Grenzwert in dem Analysefenster fällt oder ansteigt, die Aktivität des Sympathikus in der Haut beurteilt und erhalten und als erfolgreich oder normal definiert wird.

3. Verfahren nach Anspruch 2, wobei der vordefinierte Grenzwert 0,02 Mikrosiemens pro Zeit beträgt.

4. Verfahren nach Anspruch 2, wobei die Hautleitfähigkeit durch Berechnen der von den Fluktuationen betroffenen Fläche beurteilt oder gemessen wird und wobei, wenn die Fläche einen vordefinierten Grenzwert in dem Analysefenster aufweist, die Aktivität des Sympathikus in der Haut beurteilt und erhalten und als erfolgreich oder normal definiert wird.

5. Verfahren nach Anspruch 4, wobei der vordefinierte Grenzwert 2 Mikrosiemens pro Sekunde beträgt.

6. Verfahren nach Anspruch 4, wobei die Hautleitfähigkeit durch Berechnen der Amplitude der Fluktuationen der Hautleitfähigkeit beurteilt oder gemessen wird und wobei, wenn die mittlere Amplitude der Fluktuationen der Hautleitfähigkeit einen vordefinierten Grenzwert in dem Analysefenster aufweist, die Aktivität des Sympathikus in der Haut beurteilt und erhalten und als erfolgreich oder normal definiert wird.

7. Verfahren nach Anspruch 6, wobei der vordefinierte Grenzwert 0,02 Mikrosiemens beträgt.

8. Verfahren nach einem der Ansprüche 1-7, wobei die Länge des Analysefensters 15 bis 60 Sekunden beträgt.

9. Verfahren nach Anspruch 8, wobei die Länge des Analysefensters etwa 15 Sekunden beträgt.

10. Verfahren nach Anspruch 1, wobei die Hautleitfähigkeit für einen ganzen Körper des Subjekts gemessen wird.

11. Verfahren nach Anspruch 1, wobei das Subjekt ein Tier ist.

12. Verfahren nach Anspruch 1, wobei das Subjekt ein Mensch ist.

13. Verfahren nach Anspruch 1, wobei die normale Aktivität des Sympathikus in der Haut sich auf eine gemischte Nervenaktivität bezieht, bei der alle oder einige der Variablen der Hautleitfähigkeit ausgewählt werden aus der Gruppe bestehend aus Fluktuationen der Hautleitfähigkeit pro Sekunde, Amplitude der Fluktuationen der Hautleitfähigkeit, von den Fluktuationen der Hautleitfähigkeit betroffene Fläche und Änderungen in der Anstiegszeit des mittleren Hautleitfähigkeitsniveaus.

14. Verfahren nach Anspruch 1, wobei die normale Aktivität des Sympathikus in der Haut erhalten wird, wenn die Nerven nicht durch Verletzungen, Toxizität oder nervenhemmende Mittel beeinträchtigt sind.

15. Verfahren nach Anspruch 1, wobei der normale Sympathikus in der Haut auf Hautebene des Subjekts beurteilt oder gemessen wird.

16. Verfahren nach Anspruch 1, wobei die normale Aktivität des Sympathikus in der Haut an den Gliedmaßen des Subjekts, einer palmaren Seite des Handgelenks des Subjekts, der Handfläche des Subjekts, im Sprunggelenkbereich des Subjekts oder an einem plantaren Teil des Fußes des Subjekts beurteilt oder gemessen wird.

17. Verfahren nach Anspruch 1, ferner umfassend die Verwendung eines zusätzlichen Prozesses zum Beurteilen oder Messen der normalen Aktivität des Sympathikus in der Haut, wobei der zusätzliche Prozess ausgewählt wird aus der Gruppe bestehend aus unilateraler Temperaturüberwachung, bilateraler komparativer Temperaturüberwachung, Änderung in der Wellenformamplitude bei Pulsoxymetrie-Plethysmografie und einer Kombination davon.

18. Verfahren nach Anspruch 1, wobei die elektrodermale Aktivität an zwei oder mehr Gliedmaßen des Subjekts beurteilt oder gemessen wird, wobei die elektrodermale Aktivität von einer verletzten Gliedmaße oder blockierten Nerven mit der von einer oder mehreren Gliedmaße(n) ohne verletzte oder blockierte Nerven verglichen wird.

19. Verfahren nach Anspruch 1, ferner umfassend Stimulieren von elektrodermaler Aktivität in dem Subjekt, das normale Aktivität des Sympathikus in der Haut zeigt, die als erhalten oder erfolgreich beurteilt oder gemessen wird.

20. Vorrichtung, **dadurch gekennzeichnet, dass** die Vorrichtung dazu konfiguriert ist, das Verfahren nach einem der Ansprüche 1-19 durchzuführen.

21. Vorrichtung nach Anspruch 20, wobei die Vorrichtung einen drahtlosen Sensor mit Bluetooth-Verbindung zu einem Computer oder Mobiltelefon umfasst, wobei ein Signal von einer Computersoftwareanwendung verarbeitet wird und wobei die Vorrichtung drahtlose Informationen über eine Drahtlostechnologie an andere Computer oder Mobilgeräte oder Tablets mit Computersoftwareprogramm senden kann.

22. Vorrichtung nach Anspruch 20, umfassend eine Messbox mit Elektroden und Computersoftwareanzeige auf einem beliebigen Computertablet.

23. Vorrichtung nach Anspruch 20, wobei die Vorrichtung dazu konfiguriert ist, zusammen mit einem Beschleunigungsmesser verwendet zu werden, der Bewegungen mitteilt und Informationen zu Bewegungsartefakten angibt.

24. Vorrichtung nach Anspruch 20, wobei die Vorrichtung ferner dazu konfiguriert ist, einen zusätzlichen Prozess zum Beurteilen der normalen Nervenaktivität durchzuführen, wobei der zusätzliche Prozess ausgewählt wird aus der Gruppe bestehend aus unilateraler Temperaturüberwachung, bilateraler komparativer Temperaturüberwachung, Änderung in der Wellenformamplitude bei Pulsoxymetrie-Plethysmografie und einer Kombination davon.

25. Vorrichtung nach Anspruch 20, wobei die Vorrichtung dazu konfiguriert ist, die elektrodermale Aktivität an zwei oder mehr Gliedmaßen zu beurteilen, um die Gliedmaße mit normaler Aktivität des Sympathikus in der Haut mit einer oder mehreren Gliedmaßen ohne normale Aktivität des Sympathikus in der Haut zu vergleichen.

26. Vorrichtung nach Anspruch 20, wobei die Vorrichtung ferner einen Stimulator für die elektrodermale Aktivität umfasst, der dazu konfiguriert ist, Informationen dazu anzugeben, wann die normale Aktivität des Sympathikus in der Haut vorliegt, um zu definieren, dass die Nervenaktivität für die klinische Beurteilung wie Schmerzen und Aufwachen verwendet werden kann.

## Revendications

1. Procédé d'évaluation ou de surveillance de l'activité normale des nerfs sympathiques de la peau dans un sujet vivant, le sujet ayant une peau, le procédé comprenant l'évaluation ou la mesure d'une activité électrodermale,
dans lequel l'activité électrodermale évaluée ou mesurée est une conduction cutanée, la conduction cutanée est évaluée ou mesurée en calculant des pics de fluctuation de conduction cutanée par unité de temps, et
dans lequel lorsque le nombre des fluctuations de conduction cutanée est supérieur ou égal à un niveau de seuil prédéfini dans une fenêtre d'analyse l'activité des nerfs sympathiques de la peau est évaluée et obtenue et définie comme efficace ou normale,
**caractérisé en ce que** le niveau de seuil prédéfini est de 0,13 fluctuation de conduction cutanée par seconde.

2. Procédé selon la revendication 1, dans lequel la conduction cutanée est évaluée ou mesurée par un calcul du temps de montée du niveau moyen de conduction cutanée ou des fluctuations de conduction cutanée et dans lequel le temps de montée diminue ou augmente avec un niveau de seuil prédéfini dans la fenêtre d'analyse, l'activité des nerfs sympathiques de la peau est évaluée et obtenue et définie comme efficace ou normale.

3. Procédé selon la revendication 2, dans lequel le niveau de seuil prédéfini est de 0,02 microsiemens par temps.

4. Procédé selon la revendication 2, dans lequel la conduction cutanée est évaluée ou mesurée en calculant la zone sous les fluctuations et dans lequel la zone a un niveau de seuil prédéfini dans la fenêtre d'analyse, l'activité des nerfs sympathiques de la peau est évaluée et obtenue et définie comme efficace ou normale.

5. Procédé selon la revendication 4, dans lequel le seuil prédéfini est de 2 microsiemens par seconde.

6. Procédé selon la revendication 4, dans lequel la conduction cutanée est évaluée ou mesurée en calculant l'amplitude des fluctuations de conduction cutanée, et dans lequel l'amplitude moyenne des fluctuations de conduction cutanée a un niveau de seuil prédéfini dans la fenêtre d'analyse, l'activité des nerfs sympathiques de la peau est évaluée et obtenue et définie comme efficace ou normale.

7. Procédé selon la revendication 6, dans lequel le niveau de seuil prédéfini est de 0,02 microsiemens.

8. Procédé selon l'une quelconque des revendications 1 à 7 dans lequel la longueur de la fenêtre d'analyse est de 15 à 60 secondes.

9. Procédé selon la revendication 8, dans lequel la longueur de la fenêtre d'analyse est d'environ 15 secondes.

10. Procédé selon la revendication 1, dans lequel la conduction cutanée est mesurée dans un corps entier du sujet.

11. Procédé selon la revendication 1, dans lequel le sujet est un animal.

12. Procédé selon la revendication 1, dans lequel le sujet est un humain.

13. Procédé selon la revendication 1, dans lequel l'activité des nerfs sympathiques de la peau est pour une activité mixte des nerfs où toutes ou certaines des variables de conduction cutanée sont choisies dans le groupe consistant en des fluctuations de conduction cutanée par seconde, une amplitude des fluctuations de conduction cutanée, une zone sous les fluctuations de conduction cutanée et des changements d'un temps de montée du niveau moyen de conduction cutanée.

14. Procédé selon la revendication 1, dans lequel l'activité normale des nerfs sympathiques de la peau est obtenue lorsque les nerfs ne sont pas dérangés par des blessures, une toxicité ou des agences de blocage de nerfs.

15. Procédé selon la revendication 1, dans lequel les nerfs sympathiques normaux de la peau sont évalués ou mesurés à un niveau cutané du sujet.

16. Procédé selon la revendication 1, dans lequel l'activité normale des nerfs sympathiques de la peau est évaluée ou mesurée dans les membres du sujet, un côté palmaire du poignet du sujet, la paume du sujet, la zone de la cheville du sujet ou une partie plantaire du pied du sujet.

17. Procédé selon la revendication 1, comprenant en outre l'utilisation d'un procédé supplémentaire pour évaluer ou mesurer une activité normale des nerfs sympathiques de la peau, dans lequel le procédé supplémentaire est choisi dans le groupe consistant en une surveillance de thermométrie unilatérale, une surveillance de thermométrie comparative bilatérale, un changement d'amplitude de forme d'onde dans une pléthysmographie par oxymétrie du pouls, et n'importe quelle combinaison de ceux-ci.

18. Procédé selon la revendication 1, dans lequel l'activité électrodermale à deux extrémités ou plus du sujet est évaluée ou mesurée, dans lequel dans l'activité électrodermale d'une extrémité des nerfs blessés ou bloqués et une ou plusieurs extrémités sans nerfs blessés ou bloqués sont comparés.

19. Procédé selon la revendication 1, comprenant en outre la stimulation d'une activité électrodermale dans le sujet qui présente une activité normale des nerfs sympathiques de la peau est évaluée ou mesurée pour avoir été obtenue ou efficace.

20. Appareil **caractérisé en ce que** l'appareil est configuré pour réaliser le procédé selon l'une quelconque des revendications 1 à 19.

21. Appareil selon la revendication 20, dans lequel l'appareil comprend un capteur sans fil avec une connexion Bluetooth à un ordinateur ou un téléphone cellulaire dans lequel un signal est traité par le biais d'une application de logiciel informatique et dans lequel l'appareil peut envoyer des informations sans fil par le biais d'une technologie sans fil à d'autres ordinateurs, ou des dispositifs mobiles ou des tablettes avec un programme de logiciel informatique.

22. Appareil selon la revendication 20, comprenant un boîtier de mesure avec des électrodes et un affichage de logiciel informatique sur n'importe quelles tablettes informatiques.

23. Appareil selon la revendication 20, dans lequel l'appareil est configuré pour être utilisé conjointement avec un accéléromètre qui informera de mouvements pour et donner des informations sur des artéfacts de mouvements.

24. Appareil selon la revendication 20, dans lequel l'appareil est en outre configuré pour réaliser un autre procédé qui peut évaluer une activité normale des nerfs, dans lequel le procédé supplémentaire est choisi dans le groupe consistant en une surveillance de thermométrie unilatérale, une surveillance de thermométrie comparative bilatérale, un changement d'amplitude de forme d'onde dans une pléthysmographie par oxymétrie du pouls, et n'importe quelle combinaison de ceux-ci.

25. Appareil selon la revendication 20, dans lequel l'appareil est configuré pour évaluer une activité électrodermale à deux extrémités ou plus pour comparer l'extrémité avec une activité normale des nerfs sympathiques de la peau à une ou plusieurs extrémités sans activité normale des nerfs sympathiques de la peau.

26. Appareil selon la revendication 20, dans lequel l'appareil comprend en outre un stimulateur d'activité électrodermale qui est configuré pour donner des informations sur lorsque l'activité normale des nerfs sympathiques de la peau pour définir que l'activité des nerfs peut être utilisée pour une évaluation clinique telle que la douleur et l'éveil.
